# EUROPÄISCHE PATENTSCHRIFT

(11) **EP 1 480 956 B1**
(45) Veröffentlichungstag und Bekanntmachung des Hinweises auf die Patenterteilung: **23.08.2006**
(21) Anmeldenummer: 03711911.2
(22) Anmeldetag: 28.02.2003
(51) Int. Cl.: C07D 233/54, C07D 213/20, C07C 305/04, C09K 5/00, C10M 105/72

(54) **HALOGENFREIE IONISCHE FLÜSSIGKEITEN**
HALOGEN-FREE IONIC LIQUIDS
LIQUIDES IONIQUES EXEMPTS D'HALOGENES

(30) Priorität: 01.03.2002 DE 10208822
(43) Veröffentlichungstag der Anmeldung: 01.12.2004
(73) Patentinhaber: Solvent Innovation GmbH, 50829 Köln (DE)
(72) Erfinder: WASSERSCHEID, Peter, 91054 Erlangen (DE); BOESMANN, Andreas, 52068 Aachen (DE); VAN HAL, Roy, NL-6451 HD Schienveld (NL)
(74) Vertreter: Weber, Thomas
(86) Internationale Anmeldenummer: PCT/EP2003/002127
(87) Internationale Veröffentlichungsnummer: WO 2003/074494

(56) Entgegenhaltungen:
- EP-A- 0 776 880
- EP-A- 1 182 196
- EP-A- 1 182 197
- WO-A-01/81329
- WELTON T: "Room-temperature ionic liquids. Solvents for synthesis and catalysis" CHEMICAL REVIEWS, AMERICAN CHEMICAL SOCIETY. EASTON, US, Bd. 99, 1999, Seiten 2071-2083, XP002162959 ISSN: 0009-2665 in der Anmeldung erwähnt
- HUDDLESTON J G ET AL: "ROOM TEMPERATURE IONIC LIQUIDS AS NOVEL MEDIA FOR CLEAN LIQUID LIQUID EXTRACTION" CHEMICAL COMMUNICATIONS, ROYAL SOCIETY OF CHEMISTRY, GB, 1998, Seiten 1765-1766, XP002926442 ISSN: 1359-7345 in der Anmeldung erwähnt

## Beschreibung

Die Erfindung betrifft ionische Flüssigkeiten umfassend eine Verbindung der allgemeinen Formel [Kation] [R'-O-SO₃] oder [Kation] [R'-SO₃] oder Mischungen der beiden Verbindungen. Die Erfindung betrifft weiterhin Verfahren zur chemischen Umwandlung und Trennung von Stoffen umfassend die erfindungsgemäßen ionischen Flüssigkeiten als Lösungsmittel, Lösungsmittelzusätze Extraktionsmittel oder Phasentransferkatalysatoren, sowie eine Vorrichtung zum Wärmeaustausch umfassend die Ionischen Flüssigkeiten als Wärmeträger oder Wärmeträgerzusatz.

Unter ionischen Flüssigkeiten versteht man allgemein Salze oder Gemische aus Salzen, deren Schmelzpunkte unterhalb 100°C liegen (P. Wasserscheid, W. Keim, *Angew. Chem.* **2001,** *112,* 3926). Literaturbekannte Salze dieser Art bestehen aus Anionen wie z. B. Halogenostannaten, Halogenoaluminaten, Hexafluorophosphaten oder Tetrafluoroboraten kombiniert mit substituierten Ammonium-, Phosphonium, Pyridinium- oder Imidazolium-Kationen. Mehrere Veröffentlichungen beschreiben bereits die Verwendung ionischer Flüssigkeiten als Lösungsmittel für chemische Reaktionen (T. Welton, Chem. Rev. **1999**, *99*, 2071, P. Wasserscheid, W. Keim, *Angew. Chem.,* **2000**, *112*, 3926). Beispielsweise wurden Hydrierungen von Olefinen mit Rhodium(I)) (P. A. Z. Suarez, J. E. L. Dullius, S. Einloft, R. F. de Souza und J. Dupont, *Polyhedron 15*/*7,* **1996**, 1217-1219), Ruthenium(II) und Cobalt(II) komplexen (P. A. Z. Suarez, J. E. L. Dullius, S. Einloft, R. F. de Souza und J. Dupont, *Inorganica Chimica Acta 255,* **1997**, 207-209) in ionischen Flüssigkeiten mit Tetrafluoroborat-Anion erfolgreich durchgeführt. Auch die Hydroformylierung von funktionalisierten und unfunktionalisierten Olefinen gelingt mit Rhodium-Katalysatoren in ionischen Flüssigkeiten mit schwach koordinierenden Anionen (z. B. PF₆⁻, BF₄⁻) (Y. Chauvin, L. Mussmann, H. Olivier, *European Patent, EP 776880,* **1997;** Y. Chauvin, L. Mussmann, H. Olivier, *Angew. Chem., Int. Ed. Engl.,* **1995,** *34,* 2698; W. Keim, D. Vogt, H. Waffenschmidt, P. Wasserscheid, *J. of Cat.,* **1999**, *186*, 481).

Weitere wichtige Einsatzfelder ionischer Flüssigkeiten liegen in ihrer Verwendung als Extraktionsmittel zur Stofftrennung (J. G. Huddleston, H. D. Willauer, R. P. Swatloski, A. E. Visser, R. D. Rogers, *Chem. Commun.* **1998,** 1765-1766; b) A. E. Visser, R. P. Swatlowski, R. D. Rogers, *Green Chemistry* **2000,** *2(1),* 1-4) und in ihrer Verwendung als Wärmeträger (M. L. Mutch, J. S. Wilkes, *Proceedings of the Eleventh International Symposium on Molten Salts,* P. C. Trulove, H. C. De Long, G. R. Stafford and S. Deki (Hrsg.), Proceedings Volume 98-11, The Electrochemical Society, Inc, Pennington, NJ; 1998, S. 254).

Auch wenn die Definition für ionische Flüssigkeit auch solche Salze einschließt, deren Schmelzpunkte zwischen Raumtemperatur und 100°C liegen, so ist es doch für viele Anwendungen erforderlich oder wünschenswert, dass die ionischen Flüssigkeiten bereits bei Temperaturen unterhalb von Raumtemperatur flüssig sind.

Weiterhin ist für alle Anwendungen, in denen ionische Flüssigkeiten als Lösungsmittel oder Lösungsmittelzusatz im Bereich der chemischen Synthese oder Katalyse, aber auch als Wärmeträger oder als Extraktionslösungsmittel verwendet werden, eine möglichst niedrige Viskosität der ionischen Flüssigkeiten von hohem technischen Wert. Je geringer die Viskosität der ionischen Flüssigkeiten desto schneller erfolgen Diffusions- und Stofftransportvorgänge. Dies hat in den meisten Anwendungen direkte Konsequenzen für die erzielbare Raum-Zeit-Ausbeute, den Energiebedarf oder die benötigte Menge an ionischer Flüssigkeiten. Zusammenfassend läßt sich sagen, daß die Wirtschaftlichkeit fast aller Anwendungen von ionischen Flüssigkeiten wesentlich von deren Viskosität bestimmt wird, wobei die Wirtschaftlichkeit der Anwendung umso höher wird je niedriger die Viskosität der verwendeten ionischen Flüssigkeit ist.

Zahlreiche Beispiele ionischer Flüssigkeiten sind bekannt, die bei Raumtemperatur flüssig sind. Allerdings umfassen diese Systeme in der Regel Halogenidionen wie F⁻, Cl⁻, Br⁻ oder I⁻ oder solche Anionen, die Halogenatome enthalten. Typische Vertreter der letztgenannten Anionen sind - ohne Anspruch auf Vollständigkeit - [BF₄]⁻, [PF₆]⁻, [CF₃COO]⁻, [CF₃SO₃]⁻, [(CF₃SO₂)₂N]⁻, [AlCl₄]⁻, [Al₂Cl₇]⁻ oder [SnCl₃]⁻. Die Verwendung solcher Halogenatom-haltigen Anionen hat gravierende Einschränkungen für die Anwendbarkeit der entsprechenden ionischen Flüssigkeit zur Folge:
a) Die Verwendung dieser Anionen führt zu erheblichen Kosten, da selbst die Alkalisalze dieser Ionen bereits sehr teuer sind;
b) Hydrolyseprodukte der Halogenatom-haltigen Anionen führen zu erheblicher Korrosion in Stahl- und z. T. auch Glasreaktoren;
c) Die thermische Entsorgung einer "verbrauchten" ionischen Flüssigkeit mit Halogenatom-haltigen Anionen verursacht in der Regel Korrosions- und Umweltprobleme und ist daher kostspielig. Die Entsorgung über den Abbau in einer biologischen Kläranlage wird ebenfalls durch die Anwesenheit von Halogenatom-haltigen Anionen erschwert.

Generell sind daher Halogenatom-freie ionische Flüssigkeiten von besonderem technischem Interesse, und zwar besonders dann, wenn sie zusätzlich die folgende Eigenschaften aufweisen:
a) Schmelzpunkt bzw. Glaspunkt von unter 25 °C;
b) niedrige Viskosität (<0,8 Pa s@20°C (800 cPs@20°C));
c) hydrolysestabil in neutraler wäßriger Lösung (pH = 7) bis 80 °C;

Unter den nach dem Stand der Technik bekannten Halogenatom-freien ionischen Flüssigkeiten gibt es bisher keine Vertreter, die dieses komplexe technische Anforderungsprofil erfüllen kann. So sind Nitrat-, Nitrit-, Sulfat (J. S. Wilkes, M. J. Zaworotko, *J. Chem. Soc. Chem. Commun.* **1992,** 965) und Benzolsulfonatschmelzen (H. Waffenschmidt, Dissertation, RWTH Aachen 2000) zwar bekannt, diese ionischen Flüssigkeiten besitzen aber Schmelzpunkte über Raumtemperatur. Hydrogensulfate und Hydrogenphosphate reagieren in wäßriger Lösung unter Abspaltung eines oder mehrerer Protonen und bilden saure wäßrige Lösungen. Methylsulfat und Ethylsulfatschmelzen zeigen bereits nach 1h bei 80°C in wässriger Lösung deutliche Hydrolyse unter Bildung von Hydrogensulfatanionen und dem entsprechenden Alkohol (siehe auch Vergleichsbeispiele 1 und 2). EP-A-1182196 beschreibt u.a. die Herstelleung der ionische Flüssigkeit 1-Butyl-3-methylimidazolium-methylsulfat. Ionische Flüssigkeiten der allgemeinen Formel [Kation] [R-O-SO₃] besitzen eine höhere Viskosität als für die Verwendung in den meisten technischen Anwendungen gefordert (siehe Anforderung b), wenn R lediglich eine lineare oder verzweigte, gesättigte oder ungesättigte, aliphatische oder alicyclische, unfunktionalisierte oder mit einer oder mehreren Gruppen Y funktionalisierte Alkylgruppe mit 3-36 Kohlenstoffatomen darstellt und dabei Y eine -OH, -OR", -COOH, -COOR", -NR₂, -SO₄, -F, -Cl, -Br, -I oder -CN -Gruppe ist sowie R" eine verzweigte oder lineare Kohlenwasserstoffkette mit 1-12 Kohlenstoffatomen repräsentiert (siehe Vergleichsbeispiel 3).

Der vorliegenden Erfindung liegt daher die Aufgabe zugrunde halogenfreie ionische Flüssigkeiten, die einen Schmelzpunkt oder Glaspunkt von unter 25°C und Viskositäten von weniger als 0,8 Pa s@20°C (800 cPs@20°C) besitzen und verbesserte Hydrolysestabilität aufweisen bereitzustellen.

Die Aufgabe wurde erfindungsgemäß gelöst durch eine ionische Flüssigkeit umfassend eine Verbindung der der allgemeinen Formel
- [Kation] [R'-O-SO₃] oder
- [Kation] [R'-SO₃] oder
- Mischungen der beiden Verbindungen,
wobei
R' eine Gruppe der allgemeinen Formel R⁵-[X(-CH₂-)ₙ]ₘ darstellt, in der n eine Zahl zwischen 1 und 12 repräsentiert,
m eine Zahl unabhängig von n zwischen 1 und 400, vorzugsweise 50 und 300, besonders bevorzugt 100 und 200 darstellt,
X das Element Sauerstoff oder Schwefel oder eine Funktionalität der allgemeinen Form -O-Si(CH₃)₂-O-, -O-Si(CH₂CH₃)₂-O-, -O-Si(OCH₃)₂-O-, -O-Si(O-CH₂CH₃)₂-O- repräsentiert und R⁵ eine lineare oder verzweigte, gesättigte oder ungesättigte, aliphatische oder alicyclische, unfunktionalisierte oder mit einer oder mehreren Gruppen Y funktionalisierte Alkylgruppe mit 1-36 Kohlenstoffatomen darstellt wobei Y eine -OH, -OR", -COOH, -COOR", -NH₂, -SO₄, -F, -Cl, -Br, -I oder -CN -Gruppe ist und dabei R eine verzweigte oder lineare Kohlenwasserstoffkette mit 1-12 Kohlenstoffatomen repräsentiert und das verwendete [Kation] ein
- quarternäres Ammonium-Kation der allgemeinen Formel

   [NR¹R²R³R]⁺,
- Phosphonium-Kation der allgemeinen Formel

   [PR¹R² R³R]⁺,
- Imidazolium-Kation der allgemeinen Formel 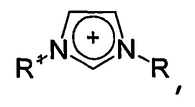 darstellt, wobei der Imidazol-Kern substituiert sein kann mit wenigstens einer Gruppe, die ausgewählt ist aus C₁₋₆-Alkyl-, C₁₋₆₋Alkoxy-, C₁₋₆-Aminoalkyl-, C₅₋₁₂-Aryl- oder C₅₋₁₂-Aryl-C₁₋₆₋Alkylgruppen,
- Pyridinium-Kationen der allgemeinen Formel 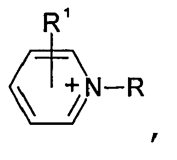 wobei der Pyridin-Kern substituiert sein kann mit wenigstens einer Gruppe, die ausgewählt ist aus C₁₋₆-Alkyl-, C₁₋₆-Alkoxy-, C₁₋₆₋Aminoalkyl-, C₅₋₁₂-Aryl- oder C₅₋₁₂-Aryl-C₁₋₆-Alkylgruppen,
- Pyrazolium-Kationen der allgemeinen Formel 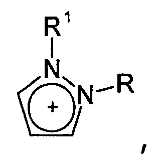 wobei der Pyrazol-Kern substituiert sein kann mit wenigstens einer Gruppe, die ausgewählt ist aus C₁₋₆-Alkyl-, C₁₋₆-Alkoxy-, C₁₋₆₋Aminoalkyl-, C₅₋₁₂-Aryl- oder C₅₋₁₂-Aryl-C₁₋₆-Alkylgruppen,
   und
- Triazolium-Kationen der allgemeinen Formel 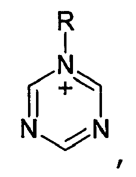 wobei der Triazol-Kern substituiert sein kann mit wenigstens einer Gruppe, die ausgewählt ist aus C₁₋₆-Alkyl-, C₁₋₆-Alkoxy-, C₁₋₆₋Aminoalkyl-, C₅₋₁₂-Aryl- oder C₅₋₁₂-Aryl-C₁₋₆-Alkylgruppen, darstellt
und die Reste R¹, R², R³ unabhängig voneinander ausgewählt sind aus der Gruppe bestehend aus
- Wasserstoff;
- linearen oder verzweigten, gesättigten oder ungesättigten, aliphatischen oder alicyclischen Alkylgruppen mit 1 bis 20 Kohlenstoffatomen;
- Oligoethylenglycolmonoalkylethern der Form [R⁴-(O-CH₂-CH₂)ₚ-O-CH₂-CH₂) wobei p eine Zahl zwischen 1 und 30 vorzugsweise zwischen 5 und 20 besonders bevorzugt zwischen 10 und 15 repräsentiert und R⁴ eine lineare oder verzweigte, gesättigte oder ungesättigte, aliphatischen oder alicyclischen Alkylgruppen mit 1 bis 20, vorzugsweise 5 bis 15, besonders bevorzugt 8 bis 12 Kohlenstoffatomen darstellt;
- Heteroaryl-, Heteroaryl-C₁₋₆-Alkylgruppen mit 3 bis 8 Kohlenstoffatomen im Heteroaryl-Rest und wenigstens einem Heteroatom ausgewählt aus N, O und S, der mit wenigstens einer Gruppe ausgewählt aus C₁₋₆-Alkylgruppen und/oder Halogenatomen substituiert sein können;
- Aryl-, Aryl-C₁₋₆-Alkylgruppen mit 5 bis 12 Kohlenstoffatomen im Arylrest, die gegebenenfalls mit wenigstens einer C₁₋₆₋Alkylgruppen und/oder einem Halogenatomen substituiert sein können;
und der Rest R ausgewählt ist aus
- linearen oder verzweigten, gesättigten oder ungesättigten, aliphatischen oder alicyclischen Alkylgruppen mit 1 bis 20, vorzugsweise 5 bis 15, besonders bevorzugt 8 bis 12 Kohlenstoffatomen;
- Oligoethylenglycolmonoalkylethern der Form [R⁴-(O-CH₂-CH₂)ₚ₋O-CH₂-CH₂) wobei p eine Zahl zwischen 1 und 30 vorzugsweise 5 und 20 besonders bevorzugt 10 und 15 repräsentiert und R⁴ eine lineare oder verzweigte, gesättigte oder ungesättigte, aliphatischen oder alicyclischen Alkylgruppen mit 1 bis 20, vorzugsweise 5 bis 15, besonders bevorzugt 8 bis 12 Kohlenstoffatomen darstellt;
- Heteroaryl-C₁₆-Alkylgruppen mit 3 bis 8 Kohlenstoffatomen im Arylrest und wenigstens einem Heteroatom ausgewählt aus N, O und S, die mit wenigstens einer C₁₋₆-Alkylgruppen und/oder Halogenatomen substituiert sein können;
- Aryl-C₁₋₆-Alkylgruppen mit 5 bis 12 Kohlenstoffatomen im Arylrest, die gegebenenfalls mit wenigstens einer C₁₋₆-Alkylgruppe und/oder einem Halogenenatomen substituiert sein können.

In einer bevorzugten Ausführungsform der vorliegenden Erfindung umfasst die ionische Flüssigkeit ein Anion der Summenformel [Me(O-CH₂-CH₂)ₙ-O-SO₃], wobei n eine Zahl von 1 bis 12, vorzugsweise 3, 4 oder 5 ist.

In einer weiteren bevorzugten Ausführungsform der Erfindung umfasst die ionische Flüssigkeit ein Anion der Summenformel [Me(O-CH₂-CH₂)ₙ₋SO₃], wobei n eine Zahlen von 1 bis 12, vorzugsweise 2 oder 3 ist.

Die erfindungsgemäßen ionischen Flüssigkeiten unterscheiden sich von den halogenfreien ionischen ionischen Flüssigkeiten des Standes der Technik durch eine deutlich erniedrigte Viskosität und sehr niedrige Schmelzpunkte. Damit erfüllen diese Materialien die oben genannte, technisch-relevante Eigenschaftskombination deutlich besser, als alle nach dem Stand der Technik bekannten ionischen Flüssigkeiten. Die erfindungsgemäßen ionischen Flüssigkeiten sind zudem hydrolysestabil in neutraler wäßriger Lösung (pH = 7) bis 80 °C. Außerdem besitzen die erfindungsgemäßen ionischen Flüssigkeiten hohe thermische Stabilitäten (bis über 250°C).

Insbesondere die erstmalig mit den ionischen Flüssigkeiten gemäß der Erfindung realisierbare Verknüpfung von halogen-freier Natur der ionischen Flüssigkeiten mit deutlich verringerter Viskosität bei gleichzeitig sehr niedrigen Schmelzpunkten macht diese neuartigen ionischen Flüssigkeiten zu idealen Substanzen für die Verwendung als Lösungsmittel bzw. Lösungsmittelzusatz für stoichiometrische oder katalytische chemische Umsetzungen sowie für ihre Anwendungen als Extraktionsmittel und/oder als Wärmeträger aus.

Weitere Ausführungsformen der vorliegenden Erfindung betreffen daher
- ein Verfahren umfassend die erfindungsgemäße ionische Flüssigkeit als Lösungsmittel, Lösungsmittelzusatz oder Phasentransfer-Katalysator. Ein solches erfindungsgemäßes Verfahren kann Übergangsmetall-, Enzym- oder mit anderen BioKatalysatoren katalysierte Reaktionsschritte umfassen, die vorzugsaweise ausgewählt sind aus Hydroformylierungsreaktionen, Oligomerisierungsreaktionen und anderen C-C-Bindungsknüpfungsreaktionen, Veresterungen, Isomerisierungsreaktionen und Reaktionen zur Amidbindungsknüpfung;
- ein Verfahren zur Stofftrennung, umfassend die erfindungsgemäße ionische Flüssigkeit als Lösungsmittel oder Lösungsmittelzusatz;
- eine Vorrichtung zum Wärmeaustausch, umfassend die erfindungsgemäße ionische Flüssigkeit als Wärmeträger oder Wärmeträgerzusatz;
- die Verwendung der erfindungsgemäßen ionischen Flüssigkeit als Lösungsmittel oder Lösungsmittelzusatz;
- die Verwendung der erfindungsgemäßen ionischen Flüssigkeit als Phasentransferkatalysator;
- die Verwendung der erfindungsgemäßen ionischen Flüssigkeit als Extraktionsmittel;
- die Verwendung der erfindungsgemäßen ionischen Flüssigkeit als Wärmeträger, sowie
- Verwendung der erfindungsgemäßen ionischen Flüssigkeit als Additiv, als oberflächenaktive Substanz, als Modifier oder als Weichmacher.

Die folgenden Verbindungen stellen weitere bevorzugte Ausführungsformen der erfindungsgemäßen ionischen Flüssigkeiten dar:
[1-Ethyl-3-methylimidazolium] [Me-(O-CH₂-CH₂)₂-O-SO₃]
[1-Ethyl-3-methylimidazolium] [Me-(O-CH₂-CH₂)₃-O-SO₃]
[1-Ethyl-3-methylimidazolium] [Me-(O-CH₂-CH₂)₄-O-SO₃]
[1-Ethyl-3-methylimidazolium] [Me-(O-CH₂-CH₂)₅-O-SO₃]
[1-Ethyl-3-methylimidazolium] [Me-(O-CH₂-CH₂)₂-SO₃]
[1-Ethyl-3-methylimidazolium] [Me-(O-CH₂-CH₂)₃-SO₃]
[1-Ethyl-3-methylimidazolium] [Me-(O-CH₂-CH₂)₂-O-SO₃]
[1-Ethyl-3-methylimidazolium] [Me-(O-CH₂-CH₂)₃-O-SO₃]
[1-Ethyl-3-methylimidazolium] [Me-(O-CH₂-CH₂)₄-O-SO₃]
[1-Ethyl-3-methylimidazolium] [Me-(O-CH₂-CH₂)₅-O-SO₃]
[1-Ethyl-3-methylimidazolium] [Me-(O-CH₂-CH₂)₂-SO₃]
[1-Ethyl-3-methylimidazolium] [Me-(O-CH₂-CH₂)₃-SO₃]
[1-Butyl-3-methylimidazolium] [Me-(O-CH₂CH₂)₂-O-SO₃]
[1-Butyl-3-methylimidazolium] [Me-(O-CH₂CH₂)₃-O-SO₃]
[1-Butyl-3-methylimidazolium] [Me-(O-CH₂-CH₂)₄-O-SO₃]
[1-Butyl-3-methylimidazolium] [Me-(O-CH₂-CH₂)₅-O-SO₃]
[1-Butyl-3-methylimidazolium] [Me-(O-CH₂-CH₂)2-SO₃]
[1-Butyl-3-methylimidazolium] [Me-(O-CH₂-CH₂)₃-SO₃]
[1-Octyl-3-methylimidazolium] [Me-(O-CH₂-CH₂)2-O-SO₃]
[1-Octyl-3-methylimidazolium] [Me-(O-CH₂-CH₂)₃-O-SO₃]
[1-Octyl-3-methylimidazolium] [Me-(O-CH₂-CH₂)₄-O-SO₃]
[1-Octyl-3-methylimidazolium] [Me-(O-CH₂-CH₂)₅-O-SO₃]
[1-Octyl-3-methylimidazolium] [Me-(O-CH₂-CH₂)₂-SO₃]
[1-Octyl-3-methylimidazolium] [Me-(O-CH₂-CH₂)₃-SO₃]
[1-Dodecyl-3-methylimidazolium] [Me-(O-CH₂-CH₂)₂-O-SO₃]
[1-Dodecyl-3-methylimidazolium] [Me-(O-CH₂-CH₂)₃-O-SO₃]
[1-Dodecyl-3-methylimidazolium] [Me-(O-CH₂-CH₂)₄-O-SO₃]
[1-Dodecyl-3-methylimidazolium] [Me-(O-CH₂-CH₂)₅-O-SO₃]
[1-Dodecyl-3-methylimidazolium] [Me-(O-CH₂-CH₂)₂-SO₃]
[1-Dodecyl-3-methylimidazolium] [Me-(O-CH₂-CH₂)₃-SO₃]
[1-Methyllmidazolium] [Me-(O-CH₂-CH₂)₂-O-SO₃]
[1-Methylimidazolium] [Me-(O-CH₂-CH₂)₃-O-SO₃]
[1-Methylimidazolium] [Me-(O-CH₂-CH₂)₄-O-SO₃]
[1-Methylimidazolium] [Me-(O-CH₂-CH₂)₅-O-SO₃]
[1-Methylimidazolium] [Me-(O-CH₂-CH₂)₂-SO₃]
[1-Methyllmidazolium] [Me-(O-CH₂-CH₂)₃-SO₃]
[Pyridinium] [Me-(O-CH₂-CH₂)₂-O-SO₃]
[Pyridinium] [Me-(O-CH₂-CH₂)₃-O-SO₃]
[Pyridinium] [Me-(O-CH₂-CH₂)₄-O-SO₃]
[Pyridinium] [Me-(O-CH₂-CH₂)₅-O-SO₃]
[Pyridinium] [Me-(O-CH₂-CH₂)₂-SO₃]
[Pyridinium] [Me-(O-CH₂-CH₂)₃-SO₃]
[1-Butylpyridinium] [Me-(O-CH₂-CH₂)₂-O-SO₃]
[1-Butylpyridinium] [Me-(O-CH₂-CH₂)₃-O-SO₃]
[1-Butylpyridinium] [Me-(O-CH₂-CH₂)₄-O-SO₃]
[1-Butylpyridinium] [Me-(O-CH₂-CH₂)₅-O-SO₃]
[1-Butylpyridinium] [Me-(O-CH₂-CH₂)₂-SO₃]
[1-Butylpyridinium] [Me-(O-CH₂-CH₂)₃-SO₃]
[Trioctylmethylammonium] [Me-(O-CH₂-CH₂)₂-O-SO₃]
[Trioctylmethylammonium] [Me-(O-CH₂-CH₂)₃-O-SO₃]
[Trioctylmethylammonium] [Me-(O-CH₂-CH₂)₄-O-SO₃]
[Trioctylmethylammonium] [Me-(O-CH₂-CH₂)₅-O-SO₃]
[Trioctylmethylammonium] [Me-(O-CH₂-CH₂)₂-SO₃]
[Trioctylmethylammonium] [Me-(O-CH₂-CH₂)₃-SO3]

Die Erfindung soll anhand der folgenden Beispiele näher erläutert werden, ohne sie jedoch auf die Beispiele zu beschränken.

### Beispiele

### Beispiel 1: [Pyridinium] [Me-(O-CH₂-CH₂)₂-O-SO₃]

### Synthese:

Zu einer Lösung von 10.48 g (87.22 mmol) Diethylenglycolmonomethylether wird bei 0°C portionsweise 13.9g Pyridin-SO₃₋Komplex (87.22 mmol) zugegeben. Die Reaktionsmischung wird bei 25°C über 18h nachgerührt. Man erhält das Produkt in quantitativer Ausbeute in Form einer gelblichen, überraschend niedrig viskosen Flüssigkeit. Der Schmelzpunkt der Substanz liegt unterhalb von 20°C.

### NMR:

¹H-NMR (300 MHz, CDCl₃): δ = 9.03 (d, 2H, CH-(CH-C**H**)₂-N), 8.71 (m, 1H, C**H**-(CH-CH)₂-N), 8.20 (m, 2H, CH-(C**H**-CH)₂-N), 4.25 (tr, 2H, O₃-SO-C**H**₂-CH₂-O-CH₂-CH₂-O-CH₃), 3.69 (tr, 2H, O₃-S-O-C**H**₂-CH₂-O-CH₂₋CH₂-O-CH₃), 3.66 (tr, 2H, O₃-S-O-CH₂-CH₂-O-C**H**₂-CH₂-O-CH₃), 3.54 (tr, 2H, O₃-S-O-CH₂-CH₂-O-CH₂-C**H**₂-O-CH₃), 3.32 (s, 3H, O₃-S-O-CH₂-CH₂₋O-CH₂-CH₂-O-C**H**₃) ppm.
¹³C-NMR (75 MHz, CDCl₃): δ = 147.1, 142.0, 127.8, 71.7, 70.1, 69.5, 66.8, 58.7 ppm.

### Beispiel 2: [Pyridinium] [Me-(O-CH₂-CH₂)₃-O-SO₃]

### Synthese:

Zu einer Lösung von 20.87 g (127.1 mmol) Triethylenglycolmonomethylether wird bei 0°C portionsweise 20.23 g Pyridin-SO₃-Komplex zugegeben (127.1 mmol). Die Reaktionsmischung wird bei 25°C über 18h nachgerührt. Man erhält das Produkt in quantitativer Ausbeute in Form einer fast farblosen Flüssigkeit. Der Schmelzpunkt der Substanz liegt unterhalb von 20°C.

### NMR:

¹H-NMR (300 MHz, CDCl₃): δ = 9.02 (d, 2H, CH-(CH-C**H**)₂-N), 8.71 (m, 1H, C**H**-(CH-CH)₂-N), 8.24 (m, 2H, CH-(C**H**-CH)₂-N), 4.25 (tr, 2H, O₃-SO-C**H**₂-CH₂-O-CH₂-CH₂-O-CH₂-CH₂-O-CH₃), 3.77 (tr, 2**H**, O₃-S-O-CH₂₋C**H**₂-O-CH₂-CH₂-O-CH₂-CH₂-O-CH₃), 3.65 (m, 6**H**, O₃-S-O-CH₂-CH₂-O-C**H**₂-C**H**₂-O-C**H**₂-CH₂-O-CH₃), 3.53 (tr, 2H, O₃-S-O-CH₂-CH₂-O-CH₂-CH₂₋O-CH₂-C**H**₂-O-CH₃), 3.32 (s, 3H, O₃-S-O-CH₂-CH₂-O-CH₂-CH₂-O-CH₂₋CH₂-O-C**H**₃) ppm.
¹³C-NMR (75 MHz, CDCl₃): δ = 147.1, 142.0, 127.8, 71.6, 70.3-70.1, 69.6, 66.8, 58.8 ppm.

### Beispiel 3: [1-Butyl-3-methylimidazolium] [Me-(O-CH₂-CH₂)₂-O-SO₃]

### Synthese:

### Methode 1:

21.88 g [Pyridinium] [Me-(O-CH₂-CH₂)₂-O-SO₃] (78.33 mmol) (dargestellt nach der unter Beispiel 1 beschriebenen Methode) und 13.68g [1-Butyl-3-methylimidazolium]Cl (78.33 mmol) werden in einer Sublimationsapparatur auf 80°C unter Vakuum erhitzt. Unter diesen Bedingungen sublimiert [Pyridinium]Cl aus der Reaktionsmischung und schlägt sich in Form weißer Nadeln an den Kühlflächen der Sublimationsapparatur nieder. Nach 8h ist das [Pyridinium]Cl vollständig aus der Reaktionmischung entfernt und das flüssige, gelbliche und überraschend niedrig viskose Produkt kann in qnatitativer Ausbeute der Sublimationsapparatur entnommen werden. Der Schmelzpunkt der Substanz liegt unterhalb von 20°C.

### Methode 2:

37.13g Diethylenglycolmonomethylether (308,99mmol) und 30.0g Sulfaminsäure (308,99mmol) werden in einen 250 ml Schlenkkolben gegeben und unter Schutzgasatmosphäre 18h unter Rühren auf 85°C erhitzt. Es bildet sich in quantitativer Ausbeute das Salz [NH₄][O₃-S-O-CH₂-CH₂-O- CH₂-CH₂-O-Me] in Form einer klaren, viskosen, gelblichen Flüssigkeit. Diese Zwischenprodukt wird bei Raumtemperatur mit 53.97g [1-Butyl-3-methylimidazolium]Cl (308,99mmol) gelöst in 300ml trockenem CH₂Cl₂ versetzt und die Mischung intensiv durchmischt. Der Niederschlag von NH₄Cl wird über eine feine Schutzgasfritte abfiltriert und das klare Filtrat eingeengt. Man erhält das Produkt mit über 95% Ausbeute in Form einer leicht gelb-bräunlichen, niedrig-viskosen Flüssigkeit. Der Schmelzpunkt der Substanz liegt unterhalb von 20°C.

### NMR:

¹H-NMR (300 MHz, CDCl₃): δ = 9.36 (s, 1H, N-C**H**-N), 7.62, 7.55 (je ein s, je 1H, N-C**H**), 4.25 (tr, 2H, N-CH₂-C**H**₂-CH₂-CH₃), 4.16 (tr, 2H, O₃-SO-C**H**₂-CH₂-O-CH₂-CH₂-O-CH₃), 4.01 (s, 3H, N-C**H**_{**3**}), 3.74 (tr, 2H, O₃-SO-CH₂-C**H**_{**2**}-O-CH₂-CH₂-O-CH₃), 3.63 (tr, 2H, O₃-S-O-CH₂-CH₂-O-C**H**_{**2-**}CH₂-O-CH₃), 3.51 (tr, 2H, O₃-S-O-CH₂-CH₂-O-C**H**_{**2**}-CH₂-O-CH₃), 3.33 (s, 3H, O₃-S-O-CH₂-CH₂-O-CH₂-CH₂-O-C**H**_{**3**}**)**, 1.87 (mult., 2H, N-CH₂-C**H**_{**2-**}CH₂-CH₃), 1.37 (mult., 2H, N-CH₂-CH₂-C**H**_{**2**}-CH₃), 0.94 (tr, 3H, N-CH₂₋CH₂-CH₂-C**H**_{**3**}) ppm.
¹³C-NMR (75 MHz, d⁶-CDCl₃): δ = 137.1, 124.0, 122.8, 71.8, 70.2, 69.9, 66.3, 58.8, 49.5, 36.2, 32.0, 19.3, 13.4 ppm.

### Viskosität

Folgende Viskositäten wurden für [1-Butyl-3-methylimidazolium] [Me-(O-CH₂-CH₂)₂-O-SO₃] bestimmt.
20°C; η= 639 cP ± 10 cP
40°C; η= 93 cP ± 3 cP
60°C; η= 64 cP ± 3 cP
80°C; η= 32 cP ± 2 cP

### Hydrolyseversuch:

5 g der ionischen Flüssigkeit [1-Butyl-3-methylimidazolium] [Me-(O-CH₂-CH₂)₂-O-SO₃] werden mit 5 ml Wasser versetzt und auf 80 °C erhitzt. Im Abstand von 10 min werden Proben aus der Reaktionslösung genommen und pH-Messungen durchgeführt. Auch nach 2h bei 80°C ist die Reaktionslösung pH-neutral, was darauf schließen läßt, daß unter diesen Reaktionsbedingungen keine hydrolytische Zersetzung der ionischen Flüssigkeit auftritt.

### Beispiel 4: [1-Octyl-3-methylimidazolium] [Me-(O-CH₂-CH₂)₂-O-SO₃]

### Synthese:

### Methode 1:

21.88 g [Pyridinium] [Me-(O-CH₂-CH₂)₂-O-SO₃] (78.33 mmol) wird in 100 ml destilliertem Wasser gelöst und portionsweise unter Kühlen mit 3.13 g NaOH (78.33 mmol) versetzt. Die wäßrige Lösung wird 15 min bei Raumtemperatur gerührt und dann dreimal mit je 200 ml Ether extrahiert.

Die wäßrige Lösung wird im Anschluß mit 21.56g [1-Octyl-3-methylimidazolium]Cl (78.33 mmol) gelöst in 100 ml Wasser vereinigt. Die wäßrige Phase wird dreimal mit je 200ml CH₂Cl₂ extrahiert, die vereinigten organischen Phasen über Na₂SO₄ getrocknet und das Lösungsmittel im Vakkum entfernt. Man erhält das Produkt mit über 95% Ausbeute in Form einer leicht gelblichen Flüssigkeit. Der Schmelzpunkt der Substanz liegt unterhalb von 20°C.

### Methode 2:

37.13g Diethylenglycolmonomethylether (308,99mmol) und 30.0g Sulfaminsäure (308,99mmol) werden in einen 250 ml Schlenkkolben gegeben und unter Schutzgasatmosphäre 18h unter Rühren auf 85°C erhitzt. Es bildet sich in quantitativer Ausbeute das Salz [NH₄][O₃-S-O-CH₂-CH₂-O- CH₂-CH₂-O-Me] in Form einer klaren, viskosen, gelblichen Flüssigkeit. Diese Zwischenprodukt wird bei Raumtemperatur in 100ml destilliertem Wasser gelöst und mit einer Lösung von 85.04g [1-Octyl-3-methylimidazolium]Cl (308,99mmol) in 100 ml Wasser vereinigt. Die wäßrige Phase wird dreimal mit je 200ml CH₂Cl₂ extrahiert, die vereinigten organischen Phasen über Na₂SO₄ getrocknet und das Lösungsmittel im Vakuum entfernt. Man erhält das Produkt mit über 95% Ausbeute in Form einer leicht gelblichen Flüssigkeit. Der Schmelzpunkt der Substanz liegt unterhalb von 20°C.

### NMR:

¹H-NMR (300 MHz, CDCl₃): δ = 9.64 (s, 1H, N-C**H**-N), 7.74, 7.60 (je ein s, je 1H, N-C**H**), 4.32 (tr, 2H, N-C**H**₂-CH₂-(CH₂)₅-CH₃), 4.18 (tr, 2H, O₃₋S-O-CH₂-C**H**₂-O-CH₂-CH₂-O-CH₃), 4.08 (s, 3H, N-C**H**₃), 3.73 (tr, 2H, O₃₋S-O-CH₂-C**H**₂-O-CH₂-CH₂-O-CH₃), 3.63 (tr, 2H, O₁₃-S-O-CH₂-CH₂-O-C**H**₂₋CH₂-O-CH₃), 3.53 (tr, 2H, O₃-S-O-CH₂-CH₂-O-CH₂-C**H**₂-O-CH₃), 3.34 (s, 3H, O₃-S-O-CH₂-CH₂-O-CH₂-CH₂-O-C**H**₃), 1.91 (tr, 2H, N-CH₂-C**H**₂₋(CH₂)₅-CH₃); 1.30 (m, 10H, N-CH₂-CH₂-(C**H**₂)₅-CH₃); 0.86 (tr, 3H, N-CH₂-CH₂-(CH₂)₅-C**H**₃) ppm.
¹³C-NMR (75 MHz, d⁶-CDCl₃): δ = 137.0, 124.0, 122.3, 71.6, 70.0, 69.8, 66.5, 58.8, 49.9, 36.4, 31.7, 30.2, 29.0, 28.9, 26.1, 22.5, 14.1 ppm.

### Hydrolyseversuch :

5 g der ionischen Flüssigkeit [1-Octyl-3-methylimidazolium] [Me-(O-CH₂-CH₂)₂-O-SO₃] werden mit 5 ml Wasser versetzt und auf 80 °C erhitzt. Im Abstand von 10 min werden Proben aus der Reaktionslösung genommen und pH-Messungen durchgeführt. Auch nach 2h bei 80°C ist die Reaktionslösung pH-neutral, was darauf schließen läßt, daß unter diesen Reaktionsbedingungen keine hydrolytische Zersetzung der ionischen Flüssigkeit auftritt.

### Vergleichsbeispiel 1: Hydrolyseversuch mit 1-n-Butyl-3-methylimidazoliummethylsulfat ([BMIM] [CH₃SO₄])

5 g der ionischen Flüssigkeit 1-n-Butyl-3-methylimidazoliummethylsulfat ([BMIM] [CH₃SO₄]) werden mit 5 ml Wasser versetzt und auf 80 °C erhitzt. Im Abstand von 10 min werden Proben aus der Reaktionslösung genommen und pH-Messungen durchgeführt. Bereits ab der ersten Messung zeigt ein rasches Absinken des pH-Werts bis auf pH 1-2. Dies läßt darauf schließen, daß unter diesen Reaktionsbedingungen eine hydrolytische Zersetzung der ionischen Flüssigkeit auftritt. Dabei wird Methanol und das saure Hydrogensulfatanion freigesetzt.

### Vergleichsbeispiel 2: Hydrolyseversuch mit 1-Ethyl-3-methylimidazoliumethylsulfat ([EMIM] [C₂H₅SO₄])

5 g der ionischen Flüssigkeit 1-Ethyl-3-methylimidazoliumethylsulfat ([EMIM] [C₂H₅SO₄]) werden mit 5 ml Wasser versetzt und auf 80 °C erhitzt. Im Abstand von 10 min werden Proben aus der Reaktionslösung genommen und pH-Messungen durchgeführt. Bereits ab der ersten Messung zeigt ein rasches Absinken des pH-Werts bis auf pH 1-2. Dies läßt darauf schließen, daß unter diesen Reaktionsbedingungen eine hydrolytische Zersetzung der ionischen Flüssigkeit auftritt. Dabei wird Ethanol und das saure Hydrogensulfatanion freigesetzt.

### Vergleichsbeispiel 3: Viskositätsmessung an der ionischen Flüssigkeit [1-Butyl-3-methylimidazolium][C₈H₁₇-O-SO₃]

Folgende Viskositäten wurden für [1-Butyl-3-methylimidazolium][C₈H₁₇₋O-SO₃] bestimmt.
20°C; η= 874 cP ± 10 cP
40°C; η= 262 cP ± 5 cP
60°C; η= 97 cP ± 3 cP
80°C; η= 46 cP ± 2 cP

### Anwendungsbeispiel 1: Rh-katalysierte Hydroformylierung von 1-Octen unter Verwendung von [1-Butyl-3-methylimidazolium] [Me-(O-CH₂-CH₂)₂-O-SO₃] als Lösungsmittel

0.05 mmol Rh(acac)(CO)2 und 0.10 mmol des Liganden NaTPPTS werden in ein Schlenkrohr eingewogen. Man gibt 5 ml der ionischen Flüssigkeit [1-Butyl-3-methylimidazolium] [Me=(O-CH2-CH2)2-O-SO3] und 5 ml Cyclohexan zu. Diese zweiphasige Lösung wird quantitativ in einen Autoklaven überführt, der mit einem kreuzförmigen Magnetrührkern bestückt wurde. Der Autoklav wird mit Synthesegas (verhältnis CO/H2= 1/1) beaufschlagt, auf 100°C erwärmt und soviel Synthesegas abgelassen, daß der Druck 30 bar beträgt. Nach einer Präformationszeit von 30 min werden 50 mmol 1-Octen über einen Tropftrichter mit Druckausgleich zugegeben. Nach 1h Reaktionszeit wird mittels Eiskühlung ein rasches Abkühlen auf Raumtemperatur erreicht und der Synthesegasdruck abgelassen. Die organische Phase wird mittels GC analysiert. Es wird 46.8% 1-Octen umgesetzt. Die Selektivität zu Aldeyden beträgt >95% was einer Katalysatoraktivität (turnoverfrequency) von 480 mol Aldehyd/mol Rh*h beträgt. Das Verhältnis der gebildeten linearen Aldehyde zu den gebildeten verzweigten Aldehyden beträgt 2.6.

### Vergleichsbeispiel zum Anwendungsbeispiel 1: Rh-katalysierte Hydroformylierung von 1-Octen unter Verwendung von [1-Butyl-3-methylimidazolium] 1C₈H₁₇-O-SO₃] als Lösungsmittel

0.05 mmol Rh(acac)(CO)2 und 0.10 mmol des Liganden NaTPPTS werden in ein Schlenkrohr eingewogen. Man gibt 5 ml der ionischen Flüssigkeit [1-Butyl-3-methylimidazolium] [C₈H₁₇-O-SO₃] und 5 ml Cyclohexan zu. Diese zweiphasige Lösung wird quantitativ in einen Autoklaven überführt, der mit einem kreuzförmigen Magnetrührkern bestückt wurde. Der Autoklav wird mit Synthesegas (Verhältnis CO/H2= 1/1) beaufschlagt, auf 100°C erwärmt und soviel Synthesegas abgelassen, daß der Druck 30 bar beträgt. Nach einer Präformationszeit von 30 min werden 50 mmol 1-Octen über einen Tropftrichter mit Druckausgleich zugegeben. Nach 1h Reaktionszeit wird mittels Eiskühlung ein rasches Abkühlen auf Raumtemperatur erreicht und der Synthesegasdruck abgelassen. Die organische Phase wird mittels GC analysiert. Es wird 15.6% 1-Octenum gesetzt. Die Selektivität zu Aldehyden beträgt >95% was einer Katalysatoraktivität (turnoverfrequency) von 160 mol Aldehyd/mol Rh*h beträgt. Das Verhältnis der gebildeten linearen Aldehyde zu den gebildeten verzweigten Aldehyden beträgt 2.6.

## Patentansprüche

1. Ionische Flüssigkeit umfassend eine Verbindung der allgemeinen Formel [Kation] [R'-O-SO₃] oder [Kation] [R'-SO₃] oder Mischungen der beiden Verbindungen,
wobei R' eine Gruppe der allgemeinen Formel R⁵-[X(-CH₂-)ₙ]ₘ darstellt, in der n eine Zahl zwischen 1 und 12 repräsentiert, m eine Zahl unabhängig von n zwischen 1 und 400, vorzugsweise 50 und 300, besonders bevorzugt 100 und 200 darstellt, X das Element Sauerstoff oder Schwefel oder eine Funktionalität der allgemeinen Form -O-Si(CH₃)₂-O-, -O-Si(CH₂CH₃)₂-O-, -O-Si(OCH₃)₂-O-, -O-Si(O-CH₂CH₃)₂-O- repräsentiert und R⁵ eine lineare oder verzweigte, gesättigte oder ungesättigte, aliphatische oder alicyclische, unfunktionalisierte oder mit einer oder mehreren Gruppen Y funktionalisierte Alkylgruppe mit 1-36 Kohlenstoffatomen darstellt wobei Y eine -OH, -OR", -COOH, -COOR", -NH₂, -SO₄, -F, -Cl, -Br, -I oder -CN -Gruppe ist und dabei R eine verzweigte oder lineare Kohlenwasserstoffkette mit 1-12 Kohlenstoffatomen repräsentiert und das verwendete [Kation] ein
- quarternäres Ammonium-Kation der allgemeinen Formel
[NR¹R²R³R]⁺,
- Phosphonium-Kation der allgemeinen Formel
[PR¹R²R³R]⁺,
- Imidazolium-Kation der allgemeinen Formel 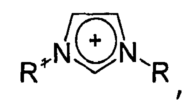 darstellt,
wobei der Imidazol-Kern substituiert sein kann mit wenigstens einer Gruppe, die ausgewählt ist aus C₁₋₆-Alkyl-, C₁₋₆-Alkoxy-, C₁₋₆₋Aminoalkyl-, C₅₋₁₂-Aryl- oder C₅₋₁₂-Aryl-C₁₋₆-Alkylgruppen,
- Pyridinium-Kationen der allgemeinen Formel 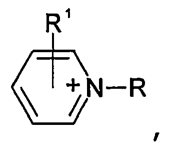 wobei der Pyridin-Kern substituiert sein kann mit wenigstens einer Gruppe, die ausgewählt ist aus C₁₋₆-Alkyl-" C₁₋₆-Alkoxy-, C₁₋₆₋Aminoalkyl-, C₅₋₁₂-Aryl- oder C₅₋₁₂-Aryl-C₁₋₆-Alkylgruppen,
- Pyrazolium-Kationen der allgemeinen Formel 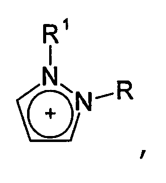 wobei der Pyrazol-Kern substituiert sein kann mit wenigstens einer Gruppe, die ausgewählt ist aus C₁₋₆-Alkyl-, C₁₋₆-Alkoxy-, C₁₋₆₋Aminoalkyl-, C₅₋₁₂-Aryl- oder C₅₋₁₂-Aryl-C₁₋₆-Alkylgruppen"
- und Triazolium-Kationen der allgemeinen Formel 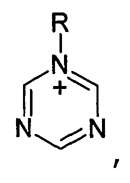 wobei der Triazol-Kern substituiert sein kann mit wenigstens einer Gruppe, die ausgewählt ist aus C₁₋₆-Alkyl-, C₁₋₆-Alkoxy-, C₁₋₆₋Aminoalkyl-, C₅₋₁₂-Aryl- oder C₅₋₁₂-Aryl-C₁₋₆-Alkylgruppen,
darstellt
und die Reste R¹, R², R³ unabhängig voneinander ausgewählt sind aus der Gruppe bestehend aus
- Wasserstoff;
- linearen oder verzweigten, gesättigten oder ungesättigten, aliphatischen oder alicyclischen Alkylgruppen mit 1 bis 20 Kohlenstoffatomen;
- Oligoethylenglycolmonoalkylethern der Form [R⁴-(O-CH₂-CH₂)ₚ₋O-CH₂-CH₂) wobei p eine Zahl zwischen 1 und 30 vorzugsweise 5 und 20 besonders bevorzugt 10 und 15 repräsentiert und R⁴ eine lineare oder verzweigte, gesättigte oder ungesättigte, aliphatischen oder alicyclischen Alkylgruppen mit 1 bis 20, vorzugsweise 5 bis 15, besonders bevorzugt 8 bis 12 Kohlenstoffatomen darstellt;
- Heteroaryl-, Heteroaryl-C₁₋₆-Alkylgruppen mit 3 bis 8 Kohlenstoffatomen im Heteroaryl-Rest und wenigstens einem Heteroatom ausgewählt aus N, O und S, der mit wenigstens einer Gruppe ausgewählt aus C₁₋₆-Alkylgruppen und/oder Halogenatomen substituiert sein können;
- Aryl-, Aryl-C₁₋₆-Alkylgruppen mit 5 bis 12 Kohlenstoffatomen im Arylrest, die gegebenenfalls mit wenigstens einer C₁₋₆₋Alkylgruppen und/oder einem Halogenatomen substituiert sein können;
und der Rest R ausgewählt ist aus
- linearen oder verzweigten, gesättigten oder ungesättigten, aliphatischen oder alicyclischen Alkylgruppen mit 1 bis 20, vorzugsweise 5 bis 15, besonders bevorzugt 8 bis 12 Kohlenstoffatomen;
- Oligoethylenglycolmonoalkylethern der Form [R⁴-(O-CH₂-CH₂)ₚ₋O-CH₂-CH₂) wobei p eine Zahl zwischen 1 und 30 vorzugsweise 5 und 20 besonders bevorzugt 10 und 15 repräsentiert und R⁴ eine lineare oder verzweigte, gesättigte oder ungesättigte, aliphatischen oder alicyclischen Alkylgruppen mit 1 bis 20, vorzugsweise 5 bis 15, besonders bevorzugt 8 bis 12 Kohlenstoffatomen darstellt;
- Heteroaryl-C₁₆-Alkylgruppen mit 3 bis 8 Kohlenstoffatomen im Arylrest und wenigstens einem Heteroatom ausgewählt aus N, O und S, die mit wenigstens einer C₁₋₆-Alkylgruppen und/oder Halogenatomen substituiert sein können;
- Aryl-C₁₋₆-Alkylgruppen mit 5 bis 12 Kohlenstoffatomen im Arylrest, die gegebenenfalls mit wenigstens einer C₁₋₆-Alkylgruppe und/oder einem Halogenenatomen substituiert sein können.

2. Ionische Flüssigkeit gemäß Anspruch 1 **dadurch gekennzeichnet, dass** die ionische Flüssigkeit ein Anion der Summenformel [Me(O-CH₂-CH₂)ₙ-O-SO₃] umfasst, wobei n eine Zahl von 1 bis 12 ist.

3. Ionische Flüssigkeit gemäß Anspruch 2, **dadurch gekennzeichnet, dass** n ausgewählt ist aus 3, 4, oder 5.

4. Ionische Flüssigkeiten gemäß Anspruch 1 **dadurch gekennzeichnet, dass** die ionische Flüssigkeit ein Anion der Summenformel [Me(O-CH₂-CH₂)ₙ-SO₃] umfasst, wobei n eine Zahlen von 1 bis 12 ist.

5. Ionische Flüssigkeiten gemäß Anspruch 4 **dadurch gekennzeichnet, dass** n ausgewählt ist aus 2 oder 3.

6. Verfahren umfassend eine ionische Flüssigkeit gemäß irgendeinem der Ansprüche 1-5 als Lösungsmittel, Lösungsmittelzusatz oder Phasentransfer-Katalysator.

7. Verfahren gemäß Anspruch 6, umfassend eine Übergangsmetallkatalysierte Reaktion.

8. Verfahren gemäß irgendeinem der Anspruch 6 bis 7, umfassend eine Reaktion, die ausgewählt ist aus Hydroformylierungsreaktionen, Oligomerisierungsreaktionen und andere C-C-Bindungsknüpfungsreaktionen, Veresterungen, Isomerisierungsreaktionen und Reaktionen zur Amidbindungsknüpfung.

9. Verfahren gemäß irgendeinem der Ansprüche 6 bis 8, umfassend ein Enzym oder einen anderen Biokatalysator.

10. Verfahren zur Stofftrennung, umfassend eine ionische Flüssigkeit gemäß irgendeinem der Ansprüche 1 bis 5 als Lösungsmittel oder Lösungsmittelzusatz.

11. Vorrichtung zum Wärmeaustausch, umfassend eine ionische Flüssigkeit gemäß irgendeinem der Ansprüche 1 bis 5 als Wärmeträger oder Wärmeträgerzusatz.

12. Verwendung einer ionischen Flüssigkeit gemäß irgendeinem der Ansprüchen 1 bis 5 als Lösungsmittel oder Lösungsmittelzusatz.

13. Verwendung einer ionischen Flüssigkeit gemäß Ansprüchen 1 bis 5 als Phasentransferkatalysator.

14. Verwendung einer ionischen Flüssigkeit gemäß irgendeinem der Ansprüche 1 bis 5 als Extraktionsmittel.

15. Verwendung einer ionischen Flüssigkeit gemäß irgendeinem der Ansprüche 1 bis 5 als Wärmeträger.

16. Verwendung einer ionischen Flüssigkeit gemäß irgendeinem der Ansprüche 1 bis 5 als Additiv, als oberflächenaktive Substanz, als Modifier oder als Weichmacher.

## Claims

1. An ionic liquid comprising a compound with the general formula [cation] [R'-O-SO₃] or [cation] [R'-SO₃] or mixtures of the two compounds; wherein
R' represents a group of general formula R⁵[X(-CH₂-)ₙ]ₘ in which n represents a number between 1 and 12;
m represents a number independent of n of between 1 and 400, preferably between 50 and 300, more preferably between 100 and 200;
X represents the element oxygen or sulfur or a functionality of general form -O-Si(CH₃)₂-O-, -O-Si(CH₂CH₃)₂-O-, -O-Si(OCH₃)₂-O-, -O-Si(O-CH₂CH₃)₂-O-; and
R⁵ represents a linear or branched, saturated or unsaturated, aliphatic or alicyclic alkyl group having 1-36 carbon atoms non-functionalized or functionalized with one or more groups Y, in which Y is an -OH, -OR", -COOH, -COOR", -NH₂, -SO₄, -F, -Cl, -Br, -I or -CN group, R" representing a branched or linear hydrocarbon chain with 1-12 carbon atoms; and the [cation] employed is a
- quaternary ammonium cation of general formula
[NR¹R²R³R]⁺,
- phosphonium cation of general formula
[PR¹R²R³R]⁺,
- imidazolium cation of general formula 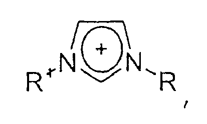 wherein the imidazole nucleus may be substituted with at least one group selected from C₁₋₆ alkyl, C₁₋₆ alkoxy, C₁₋₆ aminoalkyl, C₅₋₁₂ aryl or C₅₋₁₂-aryl-C₁₋₆-alkyl groups;
- pyridinium cations of general formula 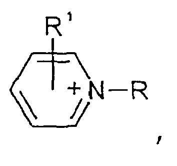 wherein the pyridine nucleus may be substituted with at least one group selected from C₁₋₆ alkyl, C₁₋₆ alkoxy, C₁₋₆ aminoalkyl, C₅₋₁₂ aryl or C₅₋₁₂-aryl-C₁₋₆-alkyl groups;
- pyrazolium cations of general formula 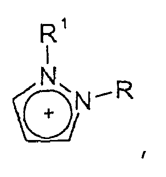 wherein the pyrazole nucleus may be substituted with at least one group selected from C₁₋₆ alkyl, C₁₋₆ alkoxy, C₁₋₆ aminoalkyl, C₅₋₁₂ aryl or C₅₋₁₂-aryl-C₁₋₆-alkyl groups; and
- triazolium cations of general formula 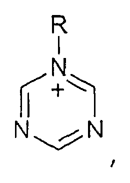 wherein the triazole nucleus may be substituted with at least one group selected from C₁₋₆ alkyl, C₁₋₆ alkoxy, C₁₋₆ aminoalkyl, C₅₋₁₂ aryl or C₅₋₁₂-aryl-C₁₋₆-alkyl groups; and
the residues R¹, R², R³ are independently selected from the group consisting of
- hydrogen;
- linear or branched, saturated or unsaturated, aliphatic or alicyclic alkyl groups having from 1 to 20 carbon atoms;
- oligoethylene glycol monoalkyl ethers of the form [R⁴-(O-CH₂-CH₂)ₚ₋O-CH₂-CH₂), wherein p represents a number of between 1 and 30, preferably between 5 and 20, more preferably between 10 and 15, and R⁴ represents a linear or branched, saturated or unsaturated, aliphatic or alicyclic alkyl group having 1 to 20, preferably 5 to 15, more preferably 8 to 12, carbon atoms;
- heteroaryl, heteroaryl-C₁₋₆-alkyl groups having from 3 to 8 carbon atoms in the heteroaryl residue and at least one heteroatom selected from N, O and S which may be substituted with at least one group selected from C₁₋₆ alkyl groups and/or halogen atoms;
- aryl, aryl-C₁₋₆-alkyl groups having from 5 to 12 carbon atoms in the aryl residue which may be optionally substituted with at least one C₁₋₆ alkyl group and/or halogen atom; and
the residue R is selected from
- linear or branched, saturated or unsaturated, aliphatic or alicyclic alkyl groups having 1 to 20, preferably 5 to 15, more preferably 8 to 12, carbon atoms;
- oligoethylene glycol monoalkyl ethers of the form [R⁴-(O-CH₂-CH₂)ₚ₋O-CH₂-CH₂), wherein p represents a number of between 1 and 30, preferably between 5 and 20, more preferably between 10 and 15, and R⁴ represents a linear or branched, saturated or unsaturated, aliphatic or alicyclic alkyl group having 1 to 20, preferably 5 to 15, more preferably 8 to 12, carbon atoms;
- heteroaryl-C₁₋₆-alkyl groups having from 3 to 8 carbon atoms in the aryl residue and at least one heteroatom selected from N, O and S which may be substituted with at least one C₁₋₆ alkyl group and/or halogen atoms;
- aryl-C₁₋₆-alkyl groups having from 5 to 12 carbon atoms in the aryl residue which may be optionally substituted with at least one C₁₋₆ alkyl group and/or halogen atom.

2. The ionic liquid according to claim 1, **characterized in that** said ionic liquid comprises an anion of formula [Me(O-CH₂-CH₂)ₙ-O-SO₃], wherein n is a number of from 1 to 12.

3. The ionic liquid according to claim 2, **characterized in that** n is selected from 3,4 or 5.

4. The ionic liquids according to claim 1, **characterized in that** said ionic liquid comprises an anion of formula [Me(O-CH₂-CH₂)ₙ-SO₃], wherein n is a number of from 1 to 12.

5. The ionic liquids according to claim 1, **characterized in that** n is selected from 2 or 3.

6. A process comprising an ionic liquid according to any of claims 1 to 5 as a solvent, solvent additive or phase-transfer catalyst.

7. The process according to claim 6, comprising a reaction catalyzed by a transition metal.

8. The process according to any of claims 6 to 7, comprising a reaction selected from hydroformylation reactions, oligomerization reactions and other C-C bond formation reactions, esterifications, isomerization reactions and reactions for amide bond formation.

9. The process according to any of claims 6 to 8, comprising an enzyme or another biocatalyst.

10. A process for the separation of materials comprising an ionic liquid according to any of claims 1 to 5 as a solvent or solvent additive.

11. A device for heat exchange comprising an ionic liquid according to any of claims 1 to 5 as a heat carrier or heat carrier additive.

12. Use of an ionic liquid according to any of claims 1 to 5 as a solvent or solvent additive.

13. Use of an ionic liquid according to any of claims 1 to 5 as a phase-transfer catalyst.

14. Use of an ionic liquid according to any of claims 1 to 5 as an extractant.

15. Use of an ionic liquid according to any of claims 1 to 5 as a heat carrier.

16. Use of an ionic liquid according to any of claims 1 to 5 as an additive, as a surface-active substance, as a modifier or as a softener.

## Revendications

1. Liquide ionique comprenant un composé de formule générale [cation] [R'-O-SO₃] ou [cation] [R'-SO₃] ou des mélanges des deux composés,
où R' représente un groupe de formule générale R⁵-[X(-CH₂-)ₙ]ₘ où n représente un nombre entre 1 et 12, m représente un nombre indépendant de n entre 1 et 400, de préférence 50 et 300, de manière particulièrement préférée 100 et 200, X représente l'élément oxygène ou soufre ou une fonctionnalité de formule générale -O-Si(CH₃)₂-O-, -O-Si(CH₂CH₃)₂-O-, -O-Si(OCH₃)₂-O-, -O-Si(O-CH₂CH₃)₂-O- et R⁵ représente un groupe alkyle ayant 1-36 atomes de carbone linéaire ou ramifié, saturé ou insaturé, aliphatique ou alicyclique, non fonctionnalisé ou fonctionnalisé avec un ou plusieurs groupe Y, où Y est un groupe -OH, -OR", -COOH, -COOR", -NH₂, -SO₄, -F, -Cl, -Br, -I ou -CN et R" représente une chaîne hydrocarbonée ramifiée ou linéaire ayant 1-12 atomes de carbone et le [cation] utilisé représente un
- cation ammonium quaternaire de formule générale
[NR¹R²R³R]⁺,
- cation phosphonium de formule générale
[PR¹R²R³R]⁺,
- cation imidazolium de formule générale 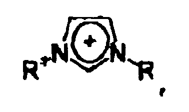 où le noyau imidazole peut être substitué avec au moins un groupe qui est choisi parmi les groupes C₁₋₆-alkyle, C₁₋₆-alcoxy, C₁₋₆-aminoalkyle, C₅₋₁₂-aryle ou C₅₋₁₂₋aryl-C₁₋₆-alkyle,
- les cations pyridinium de formule générale 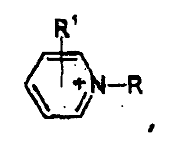 où le noyau pyridine peut être substitué avec au moins un groupe qui est choisi parmi les groupes C₁₋₆-alkyle, C₁₋₆-alcoxy, C₁₋₆-aminoalkyle, C₅₋₁₂-aryle ou C₅₋₁₂₋aryl-C₁₋₆-alkyle,
- les cations pyrazolium de formule générale 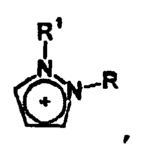 où le noyau pyrazole peut être substitué avec au moins un groupe qui est choisi parmi les groupes C₁₋₆-alkyle, C₁₋₆-alcoxy, C₁₋₆-aminoalkyle, C₅₋₁₂-aryle ou C₅₋₁₂₋aryl-C₁₋₆-alkyle,
- et les cations triazolium de formule générale 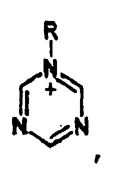 où le noyau triazole peut être substitué avec au moins un groupe qui est choisi parmi les groupes C₁₋₆-alkyle, C₁₋₆-alcoxy, C₁₋₆-aminoalkyle, C₅₋₁₂-aryle ou C₅₋₁₂₋aryl-C₁₋₆-alkyle,
et les groupements R¹, R², R³ sont choisis indépendamment les uns des autres dans le groupe consistant en
- l'hydrogène ;
- les groupes alkyle aliphatiques ou alicycliques, saturés ou insaturés, linéaires ou ramifiés, ayant 1 à 20 atomes de carbone ;
- les monoalkyléthers d'oligoéthylèneglycol de formule [R⁴-(O-CH₂-CH₂)ₚ₋O-CH₂-CH₂) où p représente un nombre entre 1 et 30 de préférence 5 et 20 de manière particulièrement préférée 10 et 15 et R⁴ représente un groupe alkyle aliphatique ou alicyclique, saturé ou insaturé, linéaire ou ramifié, ayant 1 à 20, de préférence 5 à 15, de manière particulièrement préférée 8 à 12 atomes de carbone ;
- les groupes hétéroaryle, hétéroaryl-C₁₋₆-alkyle ayant 3 à 8 atomes de carbone dans le groupement hétéroaryle et au moins un hétéroatome choisi parmi N, O et S, qui peut être substitué avec au moins un groupe choisi parmi les groupes C₁₋₆-alkyle et/ou des atomes d'halogène ;
- les groupes aryle, aryl-C₁₋₆-alkyle ayant 5 à 12 atomes de carbone dans le groupement aryle, qui peuvent éventuellement être substitués avec au moins un groupe C₁₋₆-alkyle et/ou un atome d'halogène ;
et le groupement R est choisi parmi
- les groupes alkyle aliphatiques ou alicycliques, saturés ou insaturés, linéaires ou ramifiés, ayant 1 à 20, de préférence 5 à 15, de manière particulièrement préférée 8 à 12 atomes de carbone ;
- les monoalkyléthers d'oligoéthylèneglycol de formule [R⁴-(O-CH₂-CH₂)ₚ O-CH₂-CH₂) où p représente un nombre entre 1 et 30 de préférence 5 et 20 de manière particulièrement préférée 10 et 15 et R⁴ représente un groupe alkyle aliphatique ou alicyclique, saturé ou insaturé, linéaire ou ramifié, ayant 1 à 20, de préférence 5 à 15, de manière particulièrement préférée 8 à 12 atomes de carbone ;
- les groupes hétéroaryl-C₁₆-alkyle ayant 3 à 8 atomes de carbone dans le groupement aryle et au moins un hétéroatome choisi parmi N, O et S, qui peuvent être substitués avec au moins un groupe C₁₋₆-alkyle et/ou des atomes d'halogène ;
- les groupes aryl-C₁₋₆-alkyle ayant 5 à 12 atomes de carbone dans le groupement aryle, qui peuvent éventuellement être substitués avec au moins un groupe C₁₋₆-alkyle et/ou un atome d'halogène.

2. Liquide ionique selon la revendication 1 **caractérisé en ce que** le liquide ionique comprend un anion de formule brute [Me(O-CH₂CH₂)ₙ-O-SO₃], où n est un nombre de 1 à 12.

3. Liquide ionique selon la revendication 2 **caractérisé en ce que** n est choisi parmi 3, 4 ou 5.

4. Liquides ioniques selon la revendication 1 **caractérisés en ce que** le liquide ionique comprend un anion de formule brute [Me(O-CH₂CH₂)ₙ-SO₃], où n est un nombre de 1 à 12.

5. Liquides ioniques selon la revendication 4 **caractérisés en ce que** n est choisi parmi 2 ou 3.

6. Procédé comprenant un liquide ionique selon l'une quelconque des revendications 1-5 comme solvant, solvant supplémentaire ou catalyseur de transfert de phases.

7. Procédé selon la revendication 6 comprenant une réaction catalysée par un ou des métaux de transition.

8. Procédé selon l'une quelconque des revendications 6 à 7 comprenant une réaction qui est choisie parmi les réactions d'hydroformylation, les réactions d'oligomérisation et d'autres réactions de formation de liaisons C-C, les estérifications, les réactions d'isomérisation et les réactions pour la formation de liaisons amide.

9. Procédé selon l'une quelconque des revendications 6 à 8 comprenant une enzyme ou un autre biocatalyseur.

10. Procédé pour le transfert de matière comprenant un liquide ionique selon l'une quelconque des revendications 1 à 5 comme solvant ou solvant supplémentaire.

11. Dispositif pour l'échange de chaleur comprenant un liquide ionique selon l'une quelconque des revendications 1 à 5 comme caloporteur ou caloporteur supplémentaire.

12. Utilisation d'un liquide ionique selon l'une quelconque des revendications 1 à 5 comme solvant ou solvant supplémentaire.

13. Utilisation d'un liquide ionique selon l'une quelconque des revendications 1 à 5 comme catalyseur de transfert de phases.

14. Utilisation d'un liquide ionique selon l'une quelconque des revendications 1 à 5 comme agent d'extraction.

15. Utilisation d'un liquide ionique selon l'une quelconque des revendications 1 à 5 comme caloporteur.

16. Utilisation d'un liquide ionique selon l'une quelconque des revendications 1 à 5 comme additif, comme substance tensioactive, comme modificateur ou comme plastifiant.
